# EUROPÄISCHE PATENTSCHRIFT

(11) **EP 1 658 056 B1**
(45) Veröffentlichungstag und Bekanntmachung des Hinweises auf die Patenterteilung: **07.05.2008**
(21) Anmeldenummer: 04763952.1
(22) Anmeldetag: 10.08.2004
(51) Int. Cl.: A61K 9/20

(54) **TABLETTE ENTHALTEND 3-[(2-{[4-(HEXYLOXYCARBONYLAMINO-IMINO-METHYL)-PHENYLAMINO]-METHYL}-1-METHYL-1H-BENZIMIDAZOL-5-CARBONYL)-PYRIDIN-2-YL-AMINO]-PROPIONSÄURE-ETHYLESTER ODER DESSEN SALZE**
TABLET CONTAINING 3-[(2-{[4-(HEXYLOXYCARBONYLAMINO-IMINO-METHYL)-PHENYLAMINO]- METHYL}-1-METHYL-1H-BENZIMIDAZOLO-5-CARBONYL)-PYRIDINO-2-YL- AMINO]-ETHYL PROPIONATE OR THE SALTS THEREOF
COMPRIME CONTENANT L'ETHYLESTER DE L'ACIDE 3-[(2-{[4-(HEXYLOXYCARBONYLAMINO-IMINO-METHYL)-PHENYLAMINO]-METHYL}-1-METHYL-1H-BENZIMIDAZOL-5-CARBONYL)-PYRIDIN-2-YL-AMINO]-PROPIONIQUE OU SES SELS

(30) Priorität: 16.08.2003 DE 10337697
(43) Veröffentlichungstag der Anmeldung: 24.05.2006
(73) Patentinhaber: Boehringer Ingelheim International GmbH, 55216 Ingelheim (DE); Boehringer Ingelheim Pharma GmbH & Co. KG, 55216 Ingelheim (DE)
(72) Erfinder: KOHLRAUSCH, Anja, 88400 BIBERACH (DE)
(86) Internationale Anmeldenummer: PCT/EP2004/008934
(87) Internationale Veröffentlichungsnummer: WO 2005/018615

(56) Entgegenhaltungen:
- EP-A- 0 121 901
- WO-A-03/074056
- DE-A- 10 133 786
- US-A- 4 438 091
- US-A- 6 165 507

## Beschreibung

Die Erfindung betrifft eine Tablette für den Wirkstoff 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester oder dessen pharmakologisch verträgliche Salze. Dieser Wirkstoff mit der chemischen Formel 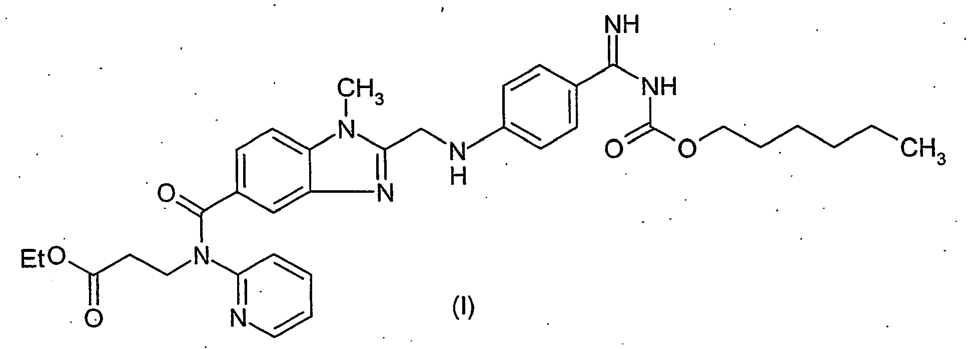 ist bereits aus der WO 98/37075, in der Verbindungen mit einer Thrombin-hemmenden und die Thrombinzeit verlängernden Wirkung offenbart werden, unter dem Namen 1-Methyl-2-[*N*-[4-(*N*-n-hexyloxycarbonylamidino)phenyl]-amino-methyl]-benzimidazol-5-yl-carbonsäure-*N*-(2-pyridyl)-*N*-(2-ethoxycarbonylethyl)-amid bekannt. Bei der Verbindung der Formel I handelt es sich um ein Doppel-Prodrug der Verbindung 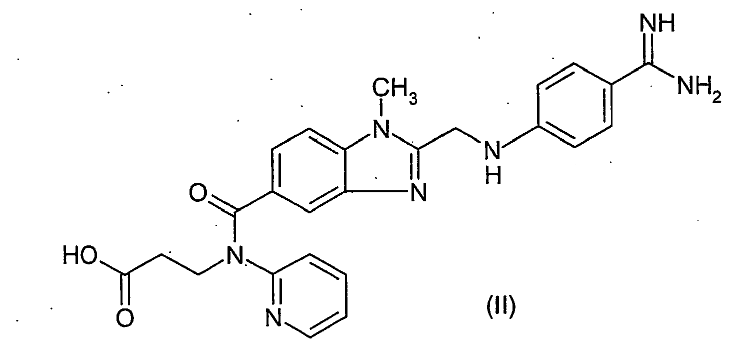 d.h. die Verbindung der Formel I wird erst im Körper in die eigentlich wirksame Verbindung, nämlich die Verbindung der Formel II, umgewandelt. Hauptindikationsgebiet der Verbindung der chemischen Formel I ist die postoperative Prophylaxe von tiefen Venenthrombosen.

aufgabe der Erfindung ist es, eine verbesserte Formulierung zur oralen Anwendung für die Verbindung der Formel (die im folgenden auch als "Wirkstoff" bezeichnet wird) bereitzustellen.

Überraschenderweise wurde nun gefunden, dass der Einsatz pharmazeutisch akzeptabler organischer Säuren mit einer Wasserlöslichkeit von > 1 g / 250 ml bei 20° C, vorzugsweise von > 1 g / 160 ml bei 25 °C, in festen oralen Darreichungsformen zu einer deutlich verbesserten galenischen Form von 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester sowie von dessen pharmazeutisch verträglichen Salzen führt.

Pharmazeutisch geeignete Säuren im Sinne dieser Erfindung sind beispielsweise Weinsäure, Fumarsäure, Bernsteinsäure, Zitronensäure und Äpfelsäure einschließlich deren Hydraten und sauren Salzen. Besonders geeignet im Sinne dieser Erfindung ist Fumarsäure.

Ausführungsform der Erfindung ist eine Tablette.

Die Tabletten enthalten 5 bis 50 Gew.-% Wirkstoff (bezogen auf das Methansulfonat), 5 bis 50 Gew.-% einer pharmazeutisch akzeptablen organischen Säure mit einer Wasserlöslichkeit von > 1 g / 250 ml bei 20° C sowie weitere Hilfs- und Füllstoffe. Als weitere Hilfs- und Füllstoffe können beispielsweise 1 bis 80 Gew.-% eines Füllstoffs, gegebenenfalls bis zu 10 Gew.-% eines Bindemittels (d.h. 0 bis 10 Gew.-% Bindemittel), 1 bis 10 Gew.-% eines Zerfallsförderers und 0,25 bis 10 Gew.-% eines Schmiermittels verwendet werden, wobei sich die Summe aller Bestandteile zu 100 Gew.-% ergänzt.

Bevorzugt sind Tabletten, die 10 bis 30 Gew.-% Wirkstoff (bezogen auf das Methansulfonat), 10 bis 40 Gew.-% einer pharmazeutisch akzeptablen organischen Säure, 5 bis 70 Gew.-% eines Füllstoffs, 3 bis 5 Gew.-% eines Bindemittels, 2 bis 6 Gew.-% eines Zerfallsförderers und 1 bis 5 Gew.-% eines Schmiermittels enthalten.

Besonders bevorzugt sind Tabletten, die 15 bis 25 Gew.-% Wirkstoff (bezogen auf das Methansulfonat), 10 bis 30 Gew.-% einer pharmazeutisch akzeptablen organischen Säure, 50 bis 65 Gew.-% eines Füllstoffs, 3 bis 5 Gew.-% eines Zerfallsförderers und 1,5 bis 2,5 Gew.-% eines Schmiermittels enthalten.

Als saurer Bestandteil kann eine pharmazeutisch akzeptable organische Säure mit einer Wasserlöslichkeit von > 1 g / 250 ml bei 20° C, wie z.B. Weinsäure, Fumarsäure, Bernsteinsäure, Zitronensäure und Äpfelsäure einschließlich deren Hydraten und sauren Salzen, verwendet werden. Als pharmazeutisch akzeptable organische Säure werden bevorzugt Weinsäure, Fumarsäure, Bernsteinsäure oder Zitronensäure eingesetzt; besonders bevorzugt ist Fumarsäure.

Unter Wirkstoff ist die Verbindung der Formel I oder eines ihrer pharmazeutisch verträglichen Salze zu verstehen. Bevorzugt ist das Methansulfonat (Mesylat) der Verbindung der Formel I.

Bei den oben erwähnten Füllstoffen, Bindemitteln, Zerfallsförderern und Schmiermitteln handelt es sich um bekannte und in der pharmazeutischen Industrie üblicherweise verwendete Verbindungen mit den genannten Eigenschaften.

Bevorzugt kommen als Füllstoff Mannitol, Erythritol, Lactose, mikrokristalline Cellulose, Hydroxypropylcellulose, insbesondere niedrigsubstiuierte Hydroxypropylcellulose, und vorgelatinierte Stärke in Frage. Besonders bevorzugt wird Mannitol verwendet.

Als Bindemittel können bevorzugterweise ein partial- oder vollsynthetischem Polymer aus der Gruppe der Polyvinylpyrrolidone (Povidone) oder der Copolymerisate aus N-Vinylpyrrolidon und Vinylacetat (Copovidone) oder Hydroxypropylmethylcellulose verwendet werden.

Als bevorzugte Zerfallsförderer kommen quervernetztes Polyvinylpyrrolidon (Crospovidone), Natriumstärkeglykolat oder quervernetztes Cellulosecarboxymethylether Natriumsalz (Croscarmellose Natrium) in Betracht. Besonders bevorzugt is Crospovidone.

Bevorzugte Schmiermittel sind beispielsweise Magnesiumstearat, Natriumstearylfumarat und Saccharosefettsäureester. Besonders bevorzugt ist Magnesiumstearat.

Die Tabletten können nach dem im folgenden beschriebenen Verfahren hergestellt werden:

### Herstellung der Tabletten

Die erfindungsgemäße Tablette kann durch direktes Mischen und Verpressen der Bestandteile oder durch Trockengranulation und Verpressen hergestellt werden. Zur Herstellung der erfindungsgemäßen Tablette kann beispielsweise wie nachfolgend beschrieben vorgegangen werden.

Der Wirkstoff, die Säure und eine Füllstoff, z.B. Mannitol werden in einem Intensivmischer vorgemischt und anschließend gesiebt. Die Pulvermischung wird in einen Freifallmischer überführt, ein Zerfallsförderer, z.B. Crospovidone und gegebenenfalls weitere Hilfstoffe (z.B. ein Bindemittel, falls erforderlich) zugesetzt und anschließend gemischt. Nach Zugabe von Schmiermitteln, insbesondere Magnesiumstearat und Saccharosefettsäureester, wird erneut gemischt. Das so erhaltene Wirkstoff-Hilfstoffgemisch wird anschließend auf einer geeigneten Tablettenpresse zu den erfindungsgemäßen Tabletten verpresst.

Der Wirkstoffgehalt der pharmazeutischen Zusammensetzung beträgt 5 bis 50 %, vorzugsweise 10 bis 30 %; der Gehalt der pharmazeutisch akzeptablen organischen Säure liegt üblicherweise zwischen 5 und 50 %, vorzugsweise zwischen 10 und 40 %.

Soweit nicht anders vermerkt, handelt es sich bei den Prozentangaben stets um Gewichtsprozente.

Bei den ersten Probanden-Versuchen mit konventionellen Tabletten enthaltend die Verbindung der Formel I war festgestellt worden, daß hochvariable Plasmaspiegel bis hin zu vereinzelten Malabsorptionen auftraten. Die Variabilität der Plasmaspiegelverläufe ist nach Applikation der Verbindung der Formel I als oral applizierte Lösung deutlich niedriger; eine Malabsorption wurde hierbei nicht beobachtet.

Ein Vorteil der erfindungsgemäßen Darreichungsform enthaltend die Verbindung der Formel I ist es, eine ausreichende, gegenüber einer konventionellen pharmazeutischen Zubereitung verbesserte sowie vom Magen-pH weitgehend unabhängige Bioverfügbarkeit des Wirkstoffs zu gewährleisten, Schwankungen der Bioverfügbarkeit des Wirkstoffs zu vermindern und Malabsorptionen zu verhindern. Eine weitere vorteilhafte Eigenschaft der erfindungsgemäßen pharmazeutischen Zusammensetzung ist ihre Eignung für alle Patienten, also auch für solche Personen, bei denen der Magen-pH durch normale physiologische Variabilität, durch eine Krankheit oder durch eine Komedikation mit Arzneimitteln, die den Magen-pH erhöhen (z.B. Pantoprazol), erhöht ist.

Die Dosierung beträgt bei oraler Gabe zweckmäßigerweise 25 bis 300 mg der Wirkstoffbase (pro Tablette), vorzugsweise 50 bis 200 mg, besonders bevorzugt 75 bis 150 mg der Wirkstoffbase, jeweils 1 bis 2 mal täglich.

Die nachfolgenden Beispiele sollen die Erfindung näher erläutern:

### Beispiel 1

### BIBR 1048 Tabletten 50 mg

| | | |
|---|---|---|
| | mg / Tablette | % / Tablette |
| Mesylat der Verbindung der Formel I¹⁾ | 57,655 | 16,957 |
| Mannitol | 205,145 | 60,337 |
| Fumarsäure | 50,000 | 14,706 |
| Crospovidone | 13,600 | 4,000 |
| Saccharosefettsäureester | 6,800 | 2,000 |
| Magnesiumstearat | 6,800 | 2,000 |
| Gesamt | 340,000 | 100,000 |

| | | |
|---|---|---|
| 1) entspricht 50 mg der Verbindung der Formel I | | |

### Beispiel 2

### BIBR 1048 Tabletten 100 mg

| | | |
|---|---|---|
| | mg / Tablette | % / Tablette |
| Mesylat der Verbindung der Formel I¹⁾ | 115,310 | 16,957 |
| Mannitol | 410,290 | 60,337 |
| Fumarsäure | 100,000 | 14,706 |
| Crospovidone | 27,200 | 4,000 |
| Saccharosefettsäureester | 13,600 | 2,000 |
| Magnesiumstearat | 13,600 | 2,000 |
| Gesamt | 680,000 | 100,000 |

| | | |
|---|---|---|
| 1) entspricht 100 mg der Verbindung der Formel I | | |

### Beispiel 3

### BIBR 1048 Tabletten 150 mg

| | mg / Tablette | % / Tablette |
|---|---|---|
| Mesylat der Verbindung der Formel I ¹⁾ | 172,963 | 23,062 |
| Mannitol | 382,037 | 50,938 |
| Fumarsäure | 150,000 | 20,000 |
| Crospovidone | 30,000 | 4,000 |
| Natriumstearylfumarat | 15,000 | 2,000 |
| Gesamt | 750,000 | 100,000 |

| | | |
|---|---|---|
| 1) entspricht 150 mg der Verbindung der Formel I | | |

### Beispiel 4

### Herstellung von 3-[(2-{[4-(Hexyloxycarbonylamino-imino-methyl)-phenylamino]-methyl}-1-methyl-1H-benzimidazol-5-carbonyl)-pyridin-2-yl-amino]-propionsäure-ethylester - Methansulfonat

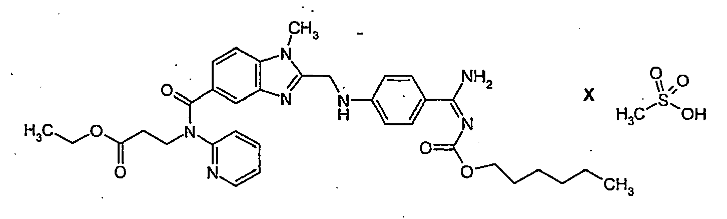

Zu einer Lösung von 3139 mg (5.0 mMol) 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester - Base (wie in der WO 98/37075 beschrieben hergestellt), in 250 ml Essigsäureethylester wurde eine Lösung von 5.0 mMol Methansulfonsäure in 25 ml Essigsäureethylester unter Rühren bei Raumtemperatur tropfenweise zugegeben. Nach wenigen Minuten begann das Produkt auszukristallisieren. Es wurde noch eine Stunde lang bei Raumtemperatur und eine weitere unter Eiskühlung gerührt, dann der Niederschlag abgesaugt, mit ca. 50 ml Essigester und 50 ml Diethylether gewaschen und bei 50°C im Umluft-Trockenschrank getrocknet. Ausbeute: 94% der Theorie
Schmelzpunkt: 178 - 179°C
C₃₄H₄₁N₇O₅ x CH₄SO₃ (723.86)

| | | | | | |
|---|---|---|---|---|---|
| Elementar-Analyse: ber.: | | C 58.07% | H 6.27% | N 13.55% | S 4.43% |
| | gef:: | 58.11% | 6.30% | 13.50% | 4.48% |

## Patentansprüche

1. Tablette umfassend
a) 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester oder eines seiner pharmazeutisch akzeptablen Salze und
b) eine oder mehrere pharmazeutisch akzeptable organische Säuren mit einer Wasserlöslichkeit von > 1 g / 250 ml bei 20° C neben üblichen Hilfs- und Füllstoffen.

2. Tablette nach Anspruch 1, bei denen die pharmazeutisch akzeptable organische Säure Weinsäure, Fumarsäure, Bernsteinsäure, Zitronensäure oder Äpfelsäure oder eines von deren Hydraten oder sauren Salzen ist.

3. Tablette nach Anspruch 2, **dadurch gekennzeichnet, dass** die pharmazeutisch akzeptable organische Säure Fumarsäure ist.

4. Tablette nach den Ansprüchen 1 bis 3, bei der der Gehalt an pharmazeutisch akzeptabler organischer Säure 5 bis 50 % beträgt.

5. Tablette nach einem der Ansprüche 1 bis 4, bei der 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester - Methansulfonat als Wirkstoff verwendet wird.

6. Tablette nach Anspruch 5, bei der der Gehalt an 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester oder dessen Salzen in der pharmazeutischen Zusammensetzung 5 bis 50 %, bezogen auf das Methansulfonat, beträgt.

## Claims

1. Tablet comprising
a) ethyl 3-[(2-{[4-(hexyloxycarbonylamino-imino-methyl)-phenylamino]-methyl}-1-methyl-1*H*-benzimidazole-5-carbonyl)-pyridin-2-yl-amino]-propionate or one of the pharmaceutically acceptable salts thereof and
b) one or more pharmaceutically acceptable organic acids with a solubility in water of > 1 g / 250 ml at 20°C together with conventional excipients and fillers.

2. Tablet according to claim 1, wherein the pharmaceutically acceptable organic acid is tartaric acid, fumaric acid, succinic acid, citric acid or malic acid or one of the hydrates or acid salts thereof.

3. Tablet according to claim 2, **characterised in that** the pharmaceutically acceptable organic acid is fumaric acid.

4. Tablet according to claims 1 to 3, wherein the content of pharmaceutically acceptable organic acid is 5 to 50 %.

5. Tablet according to one of claims 1 to 4, wherein ethyl 3-[(2-{[4-(hexyloxycarbonylamino-imino-methyl)-phenylamino]-methyl}-1-methyl-1*H*-benzimidazole-5-carbonyl)-pyridin-2-yl-amino]-propionate methanesulphonate is used as active substance.

6. Tablet according to claim 5, wherein the content of ethyl 3-[(2-{[4-(hexyloxycarbonylamino-imino-methyl)-phenylamino]-methyl}-1-methyl-1*H-*benzimidazole-5-carbonyl)-pyridin-2-yl-amino]-propionate or the salts thereof in the pharmaceutical composition is 5 to 50 %, based on the methanesulphonate.

## Revendications

1. Comprimé comprenant
a) du 3-[(2-{[4-(hexyloxycarbonylamino-imino-méthyl)-phénylamino]-méthyl}-1-méthyl-1*H*-benzimidazole-5-carbonyl)-pyridin-2-yl-amino]-propionate d'éthyle ou l'un de ses sels pharmaceutiquement acceptables et
b) un ou plusieurs acides organiques pharmaceutiquement acceptables ayant une solubilité dans l'eau > 1 g/250 ml à 20°C outre des adjuvants et charges courants.

2. Comprimé selon la revendication 1 où l'acide organique pharmaceutiquement acceptable est l'acide tartrique, l'acide fumarique, l'acide succinique, l'acide citrique ou l'acide malique ou l'un de leurs hydrates ou sels acides.

3. Comprimé selon la revendication 2 **caractérisé en ce que** l'acide organique pharmaceutiquement acceptable est l'acide fumarique.

4. Comprimé selon les revendications 1 à 3 où la teneur en acide organique pharmaceutiquement acceptable est 5 à 50 %.

5. Comprimé selon l'une des revendications 1 à 4 où le méthanesulfonate de 3-[(2-{[4-(hexyloxycarbonylamino-imino-méthyl)-phénylamino]-méthyl}-1-méthyl-1*H-*benzimidazole-5-carbonyl)-pyridin-2-yl-amino]-propionate d'éthyle est utilisé comme principe actif.

6. Comprimé selon la revendication 5 où la teneur en 3-[(2-{[4-(hexyloxycarbonylamino-imino-méthyl)-phénylamino]-méthyl}-1-méthyl-1*H-*benzimidazole-5-carbonyl)-pyridin-2-yl-amino]-propionate d'éthyle ou ses sels dans la composition pharmaceutique, rapportée au méthanesulfonate, est 5 à 50 %.
